# EUROPEAN PATENT SPECIFICATION

(11) **EP 2 393 644 B1**
(45) Date of publication and mention of the grant of the patent: **04.12.2013**
(21) Application number: 10703729.3
(22) Date of filing: 28.01.2010
(51) Int. Cl.: B29C 44/34, A61F 13/26, A61F 13/20, C08J 9/12

(54) **MICROCELLULAR INJECTION MOLDING PROCESSES FOR PERSONAL AND CONSUMER CARE PRODUCTS**
MIKROZELLULARSPRITZGIESSVERFAHREN FÜR KÖRPERPFLEGE- UND CONSUMER-CARE-PRODUKTE
PROCÉDÉS DE MOULAGE PAR INJECTION MICROCELLULAIRE DESTINÉS À DES PRODUITS DE SOINS PERSONNELS ET GRAND PUBLIC

(30) Priority: 05.02.2009 US 366318
(43) Date of publication of application: 14.12.2011
(73) Proprietor: Playtex Products, Llc, Shelton, CT 06484 (US); Wisconsin Alumni Research Foundation, Madison, WI 53726 (US)
(72) Inventor: DOUGHERTY, JR., Eugene P., Camden-Wyoming Delaware 19934 (US); EDGETT, Keith, Middletown Delaware 19709 (US); TURNG, Lih-Sheng (Tom), Madison Wisconsin 53717 (US); KRAMSCHUSTER, Adam J., Menomonie, WI 54751 (US); LEE, Jungjoo, Hillsboro, OR 97124 (US); LACEY, Chris, Cambridge Wisconsin 53523 (US); GORTON, Patrick, Clifton Park New York 12065 (US)
(74) Representative: Wihlsson, Joakim Per Magnus
(86) International application number: PCT/US2010/022314
(87) International publication number: WO 2010/090929

(56) References cited:
- EP-A1- 1 205 511
- EP-A1- 1 209 189
- EP-A2- 1 101 472
- JP-A- H0 931 328
- US-A- 5 158 986
- US-A1- 2004 198 853
- US-A1- 2004 212 118
- US-A1- 2005 042 434
- US-A1- 2006 148 917
- US-A1- 2007 148 432
- US-A1- 2008 167 597
- US-B1- 6 335 490

## Description

### Technical Field

The present invention relates, to an injection molding method for the manufacture of microcellular plastic foam for use in personal and consumer care products and packaging.

### Background of the Invention

Many personal and consumer products and packages are made of plastic. Most plastics are thermoplastics. Thermoplastics, when in solid form, melt and flow when they are heated and re-solidify upon cooling. This process is repeatable. On the other hand, some plastics are thermosetting, which means they react or crosslink under heat and pressure and set to form solids. The term "crosslink" means the attachment of two chains of polymer molecules by a bridge formed by an element, a group, or a compound that joins a carbon atom on one chain to a carbon atom on another chain by primary chemical bonds to form a crosslinking network.

Methods for processing either type of plastic, especially thermoplastics, to make personal and consumer products and packaging include injection molding, blow molding, extrusion, thermoforming, and the like. While such processes have been widely used, there are still drawbacks with these present-day processes and with the products made by these processes. For instance, while high quality injection molded articles can be produced with complex molds in large volumes at high speeds, resin costs are high, particularly for products and packages that are disposable. More recently, as oil prices increase, resin costs have been particularly high and are expected to increase still more in the future. In order to reduce overall costs which are ultimately passed to the consumer, it is therefore desirable to directly reduce the amounts of both raw materials and processing. Moreover, it would be helpful to reduce the amount of plastic waste once such products or packages are discarded into the environment.

There are, however, limits to reducing the amounts of such resin used. Mechanical properties that are indicative of the structural integrity of an article (such as modulus, stiffness, impact strength, and the like) are generally compromised if part thicknesses (and weights) are reduced beyond a certain threshold value. Articles are accordingly sized to accommodate and withstand the typical forces and pressures they are likely to be subjected to, whether in end use or during handling or transportation steps encountered in the supply chain. Articles that are too lightweight tend to bend, warp, tear, or otherwise deform under such forces and stresses.

The weight of a particular part can be reduced (and therefore the amount of resin can be reduced) by adding gas to produce a "foamed" part consisting of both air and resin. For example, "gas-assisted" injection molding processes exist in which gas is introduced into a material heated to a high temperature. By introducing gas into the heated material, resin material is displaced and the volume of the material is increased. This allows a part to have a reduced amount of resin (and lower weight) and lower cost while allowing mechanical properties to be retrained.

Unfortunately, however, gas-assisted molding processes are high in other costs and are limited mainly to thick-walled parts or parts that allow built-in thick-walled sections as gas channels. More specifically, the capabilities of gas-assisted molding processes and manufacturing tolerances associated therewith are generally insufficient for making thin-walled parts containing hollowed-out volumes that contain air or some other fill gas. Often times parts used in personal and consumer care products have thin-walled geometries. For example, petals used on tampon applicators are generally very thin, and thicknesses less than or about 0.010 inches (0.254 mm) are preferred in order to allow tampon pledgets to eject with minimal force. Hollowing out such tiny channels in thin-walled petals is very difficult as there is minimal control of the size of the air voids. Hence, even with excellent mold designs and optimized processing, there are problems with attaining good part quality, reproducible part dimensions, minimal part warpage, and shrinkage.
US2004/0212118 describes a method for producing in-mold decorated articles, which include a polymeric portion having a substrate material adhered to a surface of the polymeric portion. US 2005/0042434 describes a method of injection molding for producing a microcellular material, according to the preamble of claim 1.

### Summary of the Invention

In one aspect, the present invention resides in a method of injection molding according to claim 1. This method produces a microcellular material that can be molded into various thin-walled structures such as feminine hygiene devices. In the method, a polymer is melted and blended with a supercritical fluid to produce a single-phase polymer-gas solution. This single-phase polymer-gas solution is then injected through a nozzle and into a mold. When injected through the nozzle, gas in the polymer solution (from the supercritical fluid) emerges from the polymer solution and the polymer solidifies In emerging from the polymer solution, the gas facilitates the nucleation and subsequent growth of cells that result in a microcellular structure. The single-phase polymer-gas solution is maintained at a temperature greater than about 20 degrees C higher than the melting point of the neat polymer or plastic after the addition of the supercritical fluid and before the injection of the single-phase polymer-gas solution through the nozzle.

The method of the present invention results in a foam material. This foam material comprises a polymer having a microcellular structure formed by the nucleation and subsequent growth of micro-cells. The micro-cells are formed by the dispersing of a supercritical fluid in a liquid solution of the polymer when the polymer in liquid form is subjected to conditions of thermodynamic instability such as a sudden pressure drop that occurs during injection molding.

In another aspect, the method of the present invention resuts in a feminine hygiene device fabricated from a foamed polymer. The polymer has a microcellular structure produced by the dispersing of a superciitical fluid from the polymer in melted form when the polymer solidifies.

In another aspect, a method comprises injection molding a feminine hygiene device. In this method, a base polymer is provided to an extrusion device, for example, in pellet form and through a hopper. This base polymer may include a thermoplastic resin and at least one other additive such as a colorant, a lubricant, a slip agent, a processing aid, or the like. A supercritical fluid is also added to the extrusion device, and the base polymer and the supercritical gas are combined. The combination is then injected into a mold to provide the molded feminine hygiene device such that the hygiene device has a substantially continuous thermoplastic phase matrix.

One advantage is that the supercritical fluid aids in plasticizing the resin, thereby reducing the overall viscosity of the mix of the resin and the supercritical fluid (and also reducing the transition temperature thereof), thus allowing parts to be made using the method of the present invention at temperatures and clamp pressures that are lower than those of processes of the prior art. The lower temperatures and viscosities promote higher productivity since (1) the mix is being injected at a lower temperature with viscosities that are sufficiently low to fill the cavity and with less heat to remove, so that less cooling time is required to remove the lesser amount of heat, and (2) as the gas emerges and disperses from the mix, the resin regains its transition temperature, thereby allowing the material to vitrify (and thus harden) quickly. Moreover, the nucleation and growth of the cells of the mix effectively pack out the mold, so that the cycle time used to pack the mold is reduced or eliminated. In addition, times for cooling the part are reduced, not only because the temperature difference between the cold mold and the warmed part is reduced but also because the cell nucleation and growth is an endothermic process.

Another advantage of this process is that it can be used for a large number of different types of polymer resins. While most work to date has focused on polyamides, the microcellular foam processing process can be used for polyethylene, polypropylene, polycarbonate, polystyrene, rubber, and the like. The process can also be used for biodegradable, flushable resins such as polylactides, polyalkanoates, and even thermoplastic starch-based blends.

Another advantage is that the process allows parts manufactured thereby to be considerably reduced in weight, which means the parts manufactured can be transported more economically and that money can be saved by using less material. Furthermore, less material translates into shortened cycle times while the aesthetic and mechanical properties of the parts manufactured are comparable to those of parts manufactured by conventional and more costly processes. The weight reductions of parts manufactured by the inventive process (as compared to conventional injection molding processes) is generally about 0.5% to about 30% and preferably about 10% to about 20%.

### Brief Description of the Drawings

FIG. 1 is a schematic representation of an apparatus for carrying out the microcellular injection molding process of the present invention.
FIG. 2 is an image of a foamed polymer taken by a scanning electron microscope (SEM).
FIG. 3 is an SEM image showing cell morphology of a microcellular low-density polyethylene (LDPE) sample produced at an intermediate barrel temperature.
FIG. 4 is an SEM image showing cell morphology of a microcellular LDPE sample produced at a high barrel temperature
FIG. 5 is a photograph of two tensile bars.
FIG. 6 is a high magnification SEM image of the cell morphology of a microcellular LDPE sample.
FIG. 7 is an image of a surface profile of a conventional injection molded part taken using a Zygo^{™} Optical Profiling System (available from Zygo Corporation, Middlefield, Connecticut).
FIG. 8 is an image of a surface profile of a microcellular injection molded part taken using a Zygo^{™} Optical Profiling System (available from Zygo Corporation, Middlefield, Connecticut).
FIG. 9 is a computer screen image of a computer simulation.
FIG. 10 is a schematic representation of experiments that were performed using the computer simulation of FIG. 9.
FIG. 11 is a photograph of several tensile bars side by side obtained from the Microcellular injection molding process (150 micron teflon coating on the mold).
FIG. 12 is a photograph of several tensile bars side by side obtained from the microcellular injection molding process (75 micron teflon coating on the mold).
FIG. 13 is a side view of a petal end of a molded tampon applicator barrel part.
FIG. 14 is a cross-sectional view of a mold for a grip portion of a tampon applicator barrel part.
FIG. 15 is a photograph of a tampon applicator barrel surface produced by the process of the present invention.
FIG. 16 is a magnified image of the surface of the tampon applicator barrel of FIG. 15.
FIG. 17 is a photograph of the petals of a portion of a tampon applicator barrel produced by the process of the present invention.
FIG. 18 is an optical micrograph of the geometry of a fingergrip portion of a tampon applicator barrel produced by the process of the present invention.

### Detailed Description of the Preferred Embodiments

In one embodiment of the present invention, a microcellular injection molding process uses one or more supercritical fluids to produce foamed parts. In this process, an atmospheric gas (usually nitrogen or carbon dioxide) in a supercritical state is blended with a resin. As used herein, the term "resin" means a polymer resulting from a chemical reaction between two or more substances. The terms "resin" and "polymer" are hereinafter used interchangeably. In the present invention, the gas is blended with a polymer melt inside a machine barrel to produce a single-phase polymer-gas solution. This single-phase polymer-gas solution is injected through a nozzle and into a mold where the polymer becomes a solid. When injected through the nozzle, the sudden pressure drop that occurs causes the gas to emerge from the polymer melt to nucleate a large number (typically about 1 million to about 1 billion nucleation sites per cubic centimeter of plastic) of "micro-cells." As used herein, the term "supercritical" with regard to fluids means the highest pressure and temperature beyond which a liquid vapor transformation is not possible.

The inventive microcellular injection molding process can be used to produce various foamed parts including, but not limited to, parts for consumer and personal care products and packages. In particular, a mold and a formulation for a specified foamed part that has been designed for traditional injection molding provides a basis for rapidly and rationally developing a suitable microcellular foam process to make an improved molded part. Products that this inventive microcellular injection molding process can be utilized for include, but are not limited to, feminine hygiene devices such as tampon applicators and the packaging for such devices.

The resins that can be used in an injection molding process may be either thermoplastic or thermosetting in nature. Thermoplastic resins are preferred due to their ability to be repeatedly heated, melted, solidified and then re-melted, which allows devices into which they are incorporated to be recycled.

The use of thermosetting resins, however, is less preferred due to the crosslinking thereof, which typically inhibits the reversal and repeating of the heating, melting, and congealing processes that are characteristic of thermoplastics. When a thermosetting resin is heated, the material generally will not soften like a thermoplastic will because of the crosslinking network. Upon the application of excessive amounts of heat to a thermosetting resin, the crosslink structure can be burnt and broken. Thus, the thermosetting materials generally cannot be remelted and re-shaped after they crosslink and harden. Also, thermosetting resins are less apt to be injection molded, which is preferable for the process of the present invention, and more likely to be extruded.

The resin that is used in the process according to the present invention is low-density polyethylene (LDPE) at a concentration of at least about 70% and preferably at a concentration of at least about 80%. The LDPE is preferred because of low cost, ease of moldability, and reasonably good mechanical properties. When used in conjunction with tampons or other feminine care products, the LDPE exhibits excellent coefficient of friction values, particularly with regard to allowing for the easy and comfortable insertion of a tampon applicator. Other resins include, but are not limited to, polyamides, polypropylene, other polyolefins, blends of polyolefins and other thermoplastics, polycarbonate, polystyrene, rubber, polylactides, polyalkanoates, co- and ter-polymers comprised of the above mentioned resin types, and thermoplastic starch-based resin blends. Polylactides, polyalkanoates, and thermoplastic starch-based resin blends are renewable (sustainable), and may be flushable and/or biodegradable, thus exhibiting minimal environmental impact on waste streams.

One supercritical fluid that can be used as the atmospheric gas is nitrogen. Nitrogen is considered to be relatively inert and provides good solubility and reasonably high diffusivity in most resins. Also, nitrogen attains supercritical properties at reasonably low pressures and temperatures. For example, the critical temperature for nitrogen is 126.2 degrees K, and the critical pressure thereof is 3.39 megapascals (Mpa). Furthermore, nitrogen is currently low in cost and can be obtained with considerable ease. In one exemplary process, the loading of nitrogen as the supercritical fluid is about 0.04 weight percent (wt.%) to about 1 wt.%, with 0.05 wt.% to about 0.45 wt.% being preferred, and with 0.1 wt.% to about 0.35 wt.% being most preferred. Other supercritical fluids that could be used in this process include, but are not limited to, carbon dioxide, blends of nitrogen and carbon dioxide, and the like.

Irrespective of whether the supercritical fluid is nitrogen, carbon dioxide, or some other gas, the use of the supercritical fluid in gaseous form allows for more precise dosing and good solubility of the supercritical fluid in the resin. Nucleated cells can thus grow in a predictable, controlled fashion to reduce part weight with minimal sacrifice of mechanical properties or part aesthetics. In contrast to gas-assisted injection molding, this process makes it possible to produce the micro-cells, which are micron-sized voids, in walls as thin as 0.01 inches (0.254 mm). Mechanical properties of the final part are directly influenced by the cell size, cell density, and homogeneity of the cell structure, all of which are controllable.

Various additives may be incorporated into the resin of the microcellular injection molding process. Such additives include, but are not limited to, colorants (e.g., ultramarine blue, quinacridone violet), pigments (e.g. Pigment Yellow 180, Pigment Green 7, and the like), dyes, and opacifiers (e.g. titanium dioxide) that are used to obtain colors that are preferred by the end-user. Nacreous pigments (pearlescent materials) and/or mica may also be added to enhance aesthetic quality. Antioxidants such as hindered phenols (e.g., BHT, Irganox^{™} (Ciba) 1010, Irganox^{™} 29 and the like) and dilauryldithiopropionate can be used to aid in high temperature processing and thermal stability. Flame retardants may also be added. Lubricants, pigment dispersants and/or other processing aids (e.g., mold release agents) such as zinc stearate, erucamide, ethylene bis stearamide and the like may also be added to improve the lubricity and/or aesthetics of the material molded and, particularly with regard to feminine hygiene devices, products that are easily and comfortably inserted. Mineral fillers such as calcium carbonate can also be added to provide strength as well as to reduce overall cost. Impact modifiers and/or thermoplastic elastomers may also be added to modify impact properties and/or flexibility/elasticity.

Additionally, other additives can be incorporated into the resin to improve and to optimize the microcellular injection molding process. In particular, nanometer-sized clay particles can be added to the resin. These particles not only act to promote cell nucleation but also produce nano-composite foams having small, reproducible cell sizes. The combination of the exfoliation effects of the clay and its interfacial properties overall tends to promote cell nucleation and reduce growth rates, thereby resulting in well-dispersed, small, uniformly sized "micro-bubbles" in the foam, which provide a more stable microcellular process overall and enhanced product properties at low part weights. The resulting micro-bubbles are about 1 micron to about 200 microns in diameter and, more preferably, about 3 microns to about 20 microns. The amount of nanometer-sized clay particles added to the resin is up to about 10 wt.% with 3-5 wt.% being preferred.

Any additives incorporated into the process can be combined together into one batch (also known as a "color concentrate") and then added together with the resin to provide the desired balance of aesthetic and mechanical properties.

Blends or special grades of resin may also be useful. Alternative resins or the use of resin blends may provide advantages, which may or may not be related. The first advantage is related to viscosity, and the second is related to interfacial properties. Both tend to improve the surface quality and/or other features of the molded parts. With regard to the viscosity advantage, because the supercritical fluid used in this invention has a low viscosity, it can act as a plasticizing agent to lower the overall viscosity. The use of resins or blends that have lower viscosities than those resins used in conventional injection molding would thus be prudent. The mold would fill more quickly and the viscosity would match that of the supercritical fluid more closely. With regard to the interfacial property advantage, the use of an alternative resin or resin blend may have interfacial energy advantages. For example, the addition of small amounts of polypropylene to LDPE has been found to lower both the free energy barrier for nucleation and the interfacial energy of the resin versus the growing micro-bubbles produced in the microcellular foam process. The addition of polypropylene to LDPE can also change the crystallinity and melt strength of LDPE which can greatly affect the microcellular foamability. By matching the interfacial energy between the bubbles and resin more closely, the micro-bubbles, rather than migrating to the surface as the melt front advances and causing surface imperfections, will mix more easily with the resin to produce smooth, aesthetically pleasing parts.

Lowering the molding temperatures slightly from those that are used in conventional injection molding processes also provides some advantages. In particular, lowering the temperatures facilitates a more advantageous interfacial energy balance. Also, the lower viscosity afforded by microcellular foam molding processes allows for the molding of parts at slightly lower temperatures, which improves the energy efficiency versus that of conventional, higher temperature injection molding.

The application of a thin layer or a coating of an insulating material such as polytetrafluoroethylene (PTFE) onto the mold wall has been found to be a useful way to improve the surface quality of parts made by the processes disclosed herein. In particular, when the temperature of polymer at the mold wall is higher than the crystallization temperature of the polymer, surface defects such as swirling are reduced or eliminated. More specifically, the use of a 150 micron coating of PTFE or a fluoropolymer such as perfluoroalkoxy PFA TEFLON (hereinafter PFA) onto a thin (e.g., about 3.2 mm) cavity mold provides substantially uniform molding temperatures that achieve this result. In fact, subsequent experimentation and analyses showed that surface quality problems could be eliminated if the temperature was above the crystallization temperature at the mold wall during filling of the mold, which was achieved by the use of a thin layer of PTFE applied to the mold wall.

Furthermore, it has also been determined that the surface finish of the mold wall reflects, to a greater extent than conventional injection molding, the surface finish of the final part. Because of this, improving the finish of the mold, for example, by polishing may also dramatically improve the surface finish of the final molded part. Enhancing the surface in this manner (by polishing the surface instead of utilizing a matte finish) causes the surface to behave differently as compared to conventional injection molding processes.

The temperatures at which the microcellular injection molding process is carried out depend upon the resin used. In general, however, the temperatures at which the process is carried out are 10 to 50 centigrade degrees lower than those used in conventional injection molding processes. Actual molding temperatures can range from 10 to 60 centigrade degrees above the melting point of the polymer depending on product and mold design. The preferred reduction in temperature (versus conventional molding) is 5 to 25 centigrade degrees. In one embodiment of the present invention, the inventive microcellular injection molding process of molding tampon applicator barrels is carried out at 217 degrees centigrade. For reference, the high speed molding of LDPE-based tampon applicator barrels is typically carried out at 224 degrees centigrade. At this temperature, an increase in nucleation is expected to result from lowered surface tension, thereby reducing the activation energy barrier for cell nucleation.

Without wishing to be bound by any particular theory, it is postulated that lowering the temperature allows the presence of the supercritical fluids to reduce the viscosity of the injected melt relative to a resin formulation without a supercritical fluid injected therein, which thereby allows for operation at a lower temperature and the improvement in heat transfer efficiency. There is a limit to the lower temperature limit for this operation, however, because at some point the viscosity of the melt will become unmanageably high. Carrying out the process at around 190 degrees centigrade or greater tends to cause the nucleation of more bubbles than at lower temperatures because an increase in nucleation is expected to result from lowered surface tension, thereby reducing the activation energy barrier for cell nucleation. The cell size of the bubbles produced is about 1 micron to about 200 microns, with about 3 microns to about 20 microns being preferred.

On the other hand, very high molding temperatures can result in poor melt strength, causing the cells that do nucleate to coalescence rapidly, causing extremely large air pockets to form ("macro-bubbles") and undesirable surface features such as swirling or a gritty texture. These problems, in turn, cause poor dimensional stability, poor aesthetics, and bad color uniformity.

Cycle times for the microcellular injection molding process of the present invention are determined by mold opening and closing times, fill times, packing times, cooling times, and part ejection times. The times for various equipment movement such as times associated with closing gates in a runner system through which resin is injected as well as times for the retraction of nozzles may also have effects on the total cycle time (time from mold opening to part ejection). In the molding process, these cycle times are relatively short (compared to conventional molding processes) for disposable, high volume foamed products such as tampon application barrels. The cycle times are short for at least two reasons. First, the times needed for mold packing and holding are essentially zero. More specifically, the mold is packed as gas diffuses into the growing microcells, thus compensating for material shrinkage. Second, the heat transfer load is lessened. Based on these reasons, in manufacturing a foamed product (such as tampon application barrels) using the process, total molding cycle times of from 3 to 100 seconds are encountered. Preferred cycle times are 3 to 20 seconds, with cycle times of 4 to 15 seconds being more preferred. Results from mold viscosity tests, taken together with models for estimating the viscosity of the mix of the resin and the supercritical fluid, are used to determine an optimal fill rate (hence fill time) for a temperature of interest.

In the microcellular injection molding process, the parts of the mold that are held together to form the cavity in which the mix of the resin and the supercritical fluid is injected are clamped together. In general, due to less amount of resin being injected (and due to the lower melt viscosity owing to the presence of supercritical fluid and the low but uniform pressure resulting from cell expansion throughout the cavity that replaces the typical packing phase), the clamp pressures used in this process are less than those required for conventional injection molding. Injection pressures are lower, since clamp pressure is typically governed by injection pressure and not the packing pressure. The clamp pressures are generally up to about 50% lower than those typically used in conventional injection molding, with 10-30% lower being most preferred.

More sophisticated injection molding techniques could also be practiced. Such practices include, but are not limited to, co-injection, multi-component molding, overmolding, and the like. This would allow, for example, consumer products to be manufactured that would have different polymers having different surface characteristics. In one exemplary embodiment, one surface could have a high coefficient of friction for an easy-to-grip handle or the like, while another surface could be designed to be much smoother and lubricious and having a lower coefficient of friction.

Use of hot runner systems for molding of parts using microcellular foam injection molding is somewhat preferred. On the other hand, with hot runner systems the supercritical fluid can allow the plastic to "drool," or worse yet it can have a tendency to "propel" the plastic through the hot runner system and into the gate. It is therefore preferred to use gate valves with microcellular foam hot runner injection molding processes, so as to minimize this "drooling" or "propulsion" effect and thereby attain higher weight reduction with regard to the part being molded.

Other techniques that could be used include, but are not limited to, in-mold assembly and in-mold labeling.

In designing a particular microcellular injection molding process, the volume of parts required per year can be estimated and used together with the expected labor, overhead, packaging, material, and machine costs for producing this volume of parts along with the expected cycle time to calculate specific design parameters. Such design parameters include, but are not limited to, the number of cavities in a mold and machine tonnages. (Machine tonnage may be a design parameter consideration because the microcellular injection molding process of the present invention can utilize machines of lower tonnages due to the lower clamp pressure requirements. Also, lower machine tonnages translate into lower energy consumption values.) Any specific machine design would depend on the product requirements and other appropriate factors, such as whether or not multi-component modeling is to be performed. In this manner, once a design and process have been selected, cost quotes could be determined to assess investment criteria such as price per part requirements and overall cost savings. Computer software could be implemented to perform such calculations.

In setting parameters and in designing a suitable mold and machine, results for other similar processes can be taken together with data from suitable trials and design experiments with the microcellular foam process. This will allow for the optimization and the selection of the best combination of all parameters.

The microcellular injection molding process of the present invention is especially useful for manufacturing tampon parts such as tampon applicator barrels. Tampons are high-volume disposable products, so any attempt to reduce the environmental impact of such products is welcomed by consumers. The applicator barrels of tampons are regulated, high quality Class 2 medical devices that include very specific molded-in features such as thin-walled petals for ease of ejection of a fibrous tampon pledget as well as a much thicker, aesthetically designed grip section that is conducive to being grasped by the fingers of a user. Also, tampon applicator barrels can be manufactured efficiently and at low cost using the high speed molding processes as described herein, typically with a large number of cavities being filled rapidly and simultaneously. Furthermore, the ability to make parts from environmentally friendly resins such as polylactic acid (PLA) provides an additional benefit.

While the process of the present invention is especially suited for the injection molding of tampon applicator barrels, the overall process, methods and system described herein could be applied more widely, namely, to a wide variety of injection molded articles for personal and consumer products and packaging. In particular, microcellular foam injection molding could be used to manufacture parts for regular and disposable shaving devices, toothbrushes, battery chargers, various types of containers, bottle-top caps, toys, tampon applicator plungers (also known as pushers), and the like.

Different parameters are used for each different product made using the microcellular injection molding process. Although comparable process parameters are used for each product, when changing products, for example, when changing from tampon applicator barrels to another product, certain parameters may also need to be adjusted. For example, some products are made using a single shot injection of material whereas others are made using a multiple-step injection molding (also known as overmolding) process. When the multiple-step process is used, microcellular processing could be used in one or any number of the multiple steps.

Furthermore, other thermoplastic processing methods (e.g. film-, sheet-, and tube-extrusion, bottle blow molding, thermoforming, and the like) can be used to make consumer and personal care articles and packaging. These methods also could benefit from microcellular foam processing and the invention as described herein. Finally, even thermoset nanocomposite foams could be used to make high-strength, low weight materials used in personal and consumer care products and packaging.

### Example 1 - Tensile bar molding experiment

A standard LDPE resin was mixed together with a standard green LDPE-based batch formulation to produce a mixture containing 98.4% LDPE with the balance being inactive materials such as lubricants, slip agents, colorants, and dispersing agents. This resin formulation was used in all test specimen parts in this Example and was known as the LDPE resin mix. This resin was used in an injection molding trial to make the test specimen parts using the following experimental setup:
Injection molding machine, Arburg 320S Allrounder 55 ton (Arburg, Inc., Newington, Connecticut)
Mold: ASTM D638 tensile test bar
Coolant temperature was 38 degrees C
Super Critical Fluid injection unit from Trexel, Inc. (Woburn, Massachusetts)
Nitrogen was used as a supercritical fluid
Nitrogen injection flow rate was 0.05 kg/h (kilogram/hour) to 0.06 kg/h
Nitrogen dosage time was 1.5 seconds
Weight percentage of supercritical fluid was 0.15 to 0.17 weight percent (wt. %) (controlled by shot size)
Material was LDPE Playtex resin mix
Cycle time was about 55 seconds

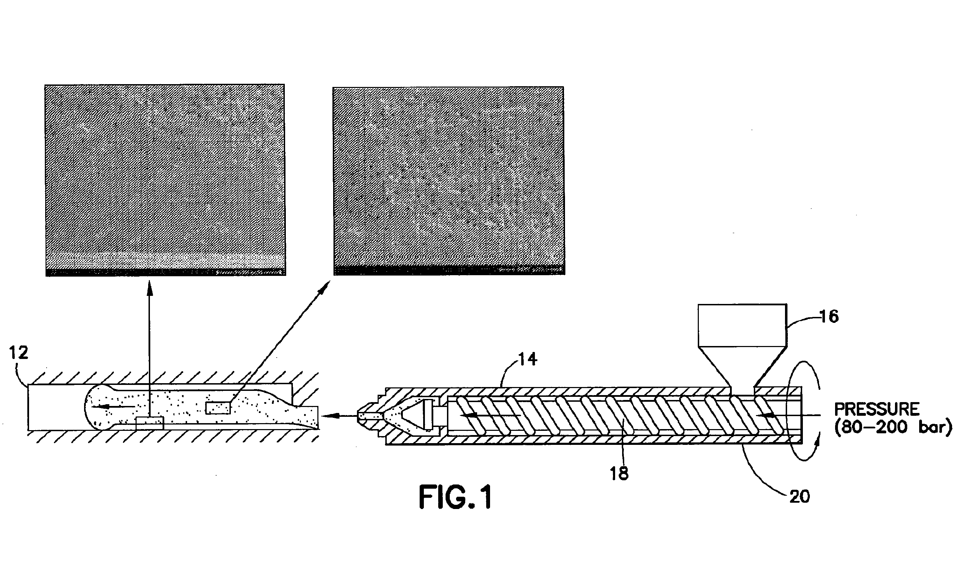

Referring to FIG. 1, an apparatus for carrying out the microcellular injection molding process is schematically shown designated generally by the reference numeral 10 and is hereinafter referred to as "apparatus 10." Apparatus 10 includes a screw conveying section 18, a hopper 16 through which the resin/colorant concentrate is added to this conveying section, and an injection molding portion 12. The screw conveying section 18, which comprises a plasticizing screw to transport the resin/colorant concentrate from the introduction thereof through the hopper 16 to the injection molding portion 20, also includes a feed system 14 for the supercritical fluid addition. The back pressure provided intermediate the screw conveying section 18 and the injection molding portion 12 is about 80 bar to about 200 bar. The resin/colorant solution is heated as it moves from the hopper 16 through the conveying section 18 using primarily the mechanical energy from the rotation of the plasticizing screw and any suitable heating means (e.g., heat from shear or heat from an electrical source) to produce a melt. When the supercritical fluid is added via the feed system 14, the resulting melt becomes a single-phase polymer-gas solution. During the filling stage, the single-phase polymer-gas solution comprising the supercritical fluid is injected into a mold via a suitable system of runners and gates. The rapid pressure drop as the solution leaves the conveying section 18 leads to the formation of nuclei and the microcellular injection molded parts having about as many as about 10⁶ to about 10⁹ pores per cubic centimeter of material. Such microcellular plastics made by this process have solid skin layers and foamed core parts.

Several different operating temperature profiles and several different shot sizes of the LDPE resin mix were used to obtain the desired reduction in part weight.

Dimensions of parts were determined using an optical comparator (Hawk Mono Dynascope, Model QC 200, available from Vision Engineerino of Bedford, New Hampshire). A commercially-available scanning electron microscope (SEM) (Model JSM-6100, available from JEOL USA, Inc. of Peabody, Massachusetts) was used to analyze tensile parts visually.

Table 1 presents data for the test specimen parts of this Example. Comparative samples are listed as C1, C2, and C3 while samples A, B, C, D, and E exemplify the microcellular injection molding process Sample C best exemplifies the microcellular injection molding process.

**Table 1 - Processes, temperatures, weights, and weight reductions. The molding temperature refers to the temperature setting at the machine nozzle.**

| Sample | Process | Molding temperature (degrees C) | Part weight (grams) | % weight reduction vs. standard process |
|---|---|---|---|---|
| C1 | Standard | 154 | 7.36 | NA |
| C2 | Standard | 134 | 7.33 | NA |
| C3 | Standard | 224 | 7.28 | NA |
| A | Microcellular | 154 | 7.23 | 1.77 |
| B | Microcellular | 134 | 7.12 | 2.86 |
| C | Microcellular | 224 | 6.11 | 16.07 |
| D | Microcellular | 224 | 5.78 | 20.60 |
| E | Microcellular | 223 | 6.58 | 9.62 |

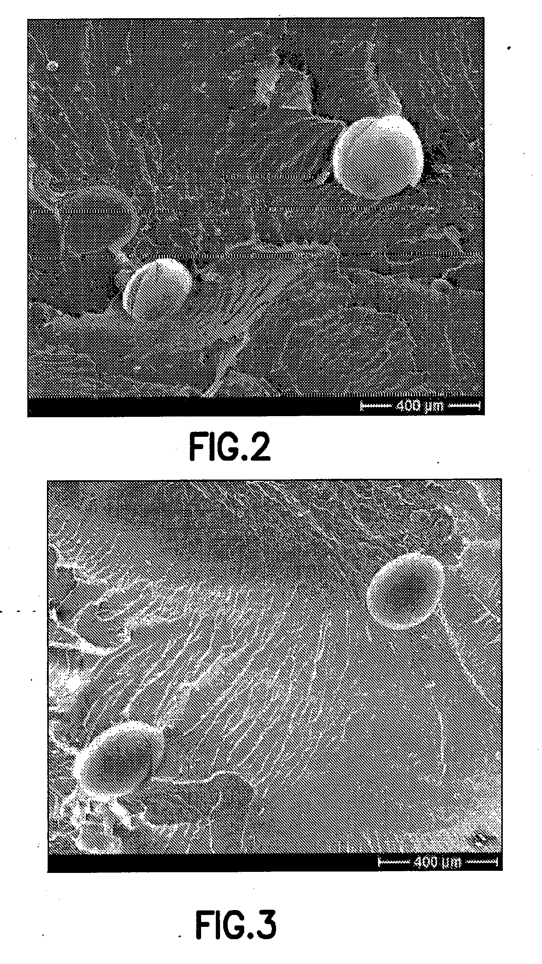

For samples B and C2 (run at the lowest molding temperature), it was noted that there were not a significant amount of bubbles in tensile bars of the microcellular-injected parts. Also, the average size of the bubbles (from the electron microscopy data) was about 200 to about 300 micrometers in diameter (as can be seen in FIG. 2, which is an image taken for a low (134 degrees C) barrel temperature and an injection shot volume of 20 cm³). Furthermore, only a few cells among the numerous nucleation sites grew at a low temperature condition.

For samples A and C1 (run at the intermediate molding temperature), it was noted that the microcellular-injected parts made under these conditions had fewer bubbles than other parts and that the bubbles were about 200 micrometers in diameter. Also, it was noted that a nozzle temperature of 151 degrees centigrade was too low to nucleate cells in polymers. Only a few bubbles were observed in the SEM graphics (as can be seen in FIG. 3, which is an image taken for a barrel temperature of 154 degrees C and an injection shot volume of 20 cm³).

Samples C, D, E, and C3 were run at the highest molding temperature. Sample E represented a 9.6% reduction in weight. For sample E, it was noted that the average cell size was around 300 to 400 micrometers, which was considered to be a large bubble as compared to other bubbles (particularly when compared to the bubbles generated at lower molding temperatures). It was postulated that these cell sizes may have been due to low melt strength, thus allowing the bubbles to grow as a result of higher melt temperatures. Furthermore, referring now to FIGS. 2-4, it was noted that microcellular injection molded LDPE samples made at a high molding temperature had more bubbles than those made at a lower barrel temperature. The observed increase in nucleation was believed to be the result of lowered surface tension, which reduced the activation energy barrier for cell nucleation.

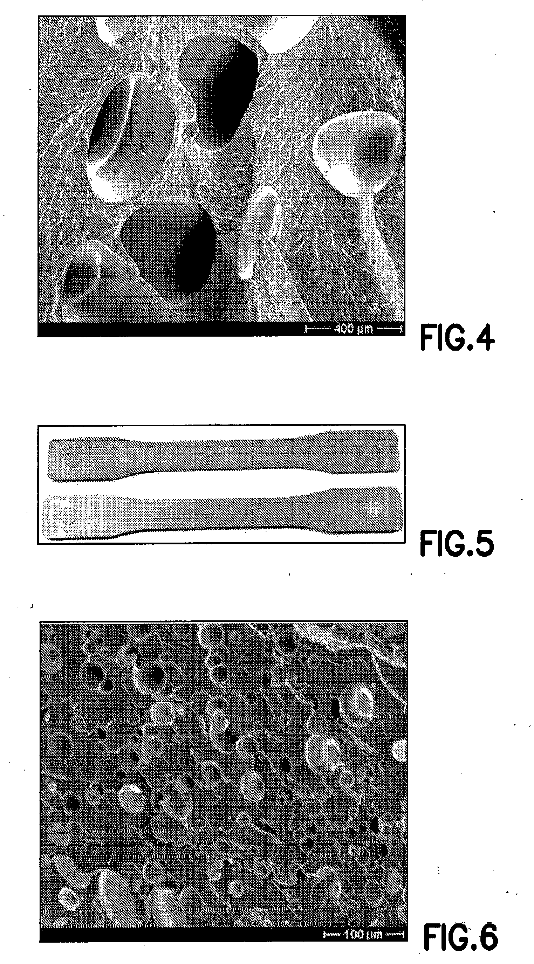

As is shown with reference to FIG. 5, an actual part 30 molded as sample C and a comparative part 32 molded as sample C3 (no microcellular foaming) were compared. Visually, the actual parts were comparable. However, the part 30 of sample C exhibited a 16% reduction in weight over the part 32 of sample C3. The part 30 of sample C also exhibited some swirling patterns that were not present in the part 32 of sample C3.

In SEM micrographs of sample C (FIG. 6), it was noted that there are numerous cells of sizes from 10 micrometers to 100 micrometers. Figure 6 shows a higher magnification image of the cell morphology of a Microcellular LDPE sample produced at a higher barrel temperature (224 deg C) and an injection shot volume of 19 cm³. Much smaller cells were seen in sample C at these experimental conditions than at other conditions. Cell sizes ranged from about 5 micrometers to about 200 micrometers.

For sample D, a weight reduction of about 21% (over sample C3) was noted. As can be seen from the SEM graphics (FIG. 4), some large gas pockets formed in the LDPE resin when it was injected. This part of the polymer was foamed with large bubbles. In FIG. 4, the high barrel temperature was 224 deg C with an injection shot volume of 20 cm³. Comparable results were obtained when the high barrel temperature was 224 deg C with an injection shot volume of 18 cm³.

Table 2 provides dimensional analysis for the samples manufactured by the process. The dimensions among the samples (i.e., lengths, widths, and thicknesses) were all fairly consistent and comparable despite the differences in temperature and part weight reduction. When the dimensions were rechecked several weeks later, the parts were found to have remained dimensionally consistent The only exception was with regard to the thickness observed at 30% from the end of the part weight for sample D. The increased thickness observed (0.1401 inches (3.55854 mm)) was likely due to a high rate of coalescence of gas bubbles.

**Table 2 - Processes, temperatures, and dimensional analysis for parts**

| | | | Lengths measured 2 times | | Widths measured at 10, 30, 50, 70, and 90% of length | | | | | Thicknesses measured at 10, 30, 50, 70, and 90% of length | | | | |
|---|---|---|---|---|---|---|---|---|---|---|---|---|---|---|
| Sample | Process | Molding temp., degrees C | L1 | L2 | W10 | W30 | W50 | W70 | W90 | T10 | T30 | T50 | T70 | T90 |
| C1 | Std. | 154 | 6.4385 | 6.4500 | 0.7403 | 0.5109 | 0.4982 | 0.5078 | 0.7493 | 0.1260 | 0.1263 | 0.1263 | 0.1269 | 0.1263 |
| C2 | Std. | 134 | 6.4400 | 6.4370 | 0.7483 | 0.5088 | 0.4974 | 0.5061 | 0.7436 | 0.1259 | 0.1259 | 0.1255 | 0.1263 | 0.1289 |
| C3 | Std. | 224 | 6.4560 | 6.4540 | 0.7424 | 0.5081 | 0.4930 | 0.5013 | 0.7408 | 0.1270 | 0.1256 | 0.1291 | 0.1281 | 0.1261 |
| A | Micro-cell. | 154 | 6.4400 | 6.4390 | 0.7480 | 0.5124 | 0.5016 | 0.5122 | 0.7515 | 0.1285 | 0.1274 | 0.1256 | 0.1254 | 0.1257 |
| B | Micro-cell | 134 | 6.4230 | 6.4295 | 0.7440 | 0.5034 | 0.4939 | 0.5052 | 0.7439 | 0.1253 | 0.1253 | 0.1263 | 0.1235 | 0.1241 |
| C | Micro-cell | 224 | 6.4505 | 6.4575 | 0.7486 | 0.5091 | 0.4980 | 0.5089 | 0.7466 | 0.1270 | 0.1258 | 0.1257 | 0.1247 | 0.1289 |
| D | Micro-cell | 224 | 6.4485 | 6.4565 | 0.7443 | 0.5071 | 0.4974 | 0.5098 | 0.7471 | 0.1270 | 0.1401 | 0.1267 | 0.1266 | 0.1275 |
| E | Micro-cell | 224 | 6.4425 | 6.4440 | 0.7521 | 0.5141 | 0.5044 | 0.5114 | 0.7480 | 0.1261 | 0.1282 | 0.1256 | 0.1293 | 0.1262 |

Table 3 provides a summary of tensile strength results for some of the samples versus control sample C3. A loss in some mechanical properties was observed. However, the loss is considered slight in view of the smaller amount of plastic being used.

**Table 3 - Tensile testing of tensile bars made by process using supercritical fluid to produce foamed parts versus conventional injection molding**

| Test Conditions | | | | | | | | | |
|---|---|---|---|---|---|---|---|---|---|
| Test speed | | 2 in/min | | | | | | | |
| Width of tensile bars | | 0.5 in | | | | | | | |
| Thickness of tensile bars | | 0.125 in | | | | | | | |
| | | | | | | | | | |

| Results | Weight % | Yield stress (psi) | | Strain at break (%) | | Modulus | | Ultimate stress (psi) | |
|---|---|---|---|---|---|---|---|---|---|
| Patent Example | (% red. (vs Ex. C3) | Average | (% red. (vs. Ex. C3) | Average | (% red. (vs. Ex. C3) | Averag e | (% red. (vs. Ex. C3) | Average | (5 red. (vs. Ex. C3) |
| C3 | 0 | 433.6 | NA | 183.0 | NA | 22.2 | NA | 1331.7 | NA |
| (comparative) | 9.62% | 373.2 | 13.9% | 119.0 | 35.0 % | 21.3 | 4.1% | 1233.0 | 7.4% |
| C | 16.07% | 314.7 | 27.4% | 107.1 | | 17.5 | 21.2% | 1043.2 | 21.7% |
| D | 20.60% | 289.9 | 33.1% | 63.8 | 41.5 % | 16.5 | 25.7% | 969.6 | 27.2% |
| E | | | | | | | | | |
| | | | | | 65.1 % | | | | |
| | | | | | | | | | |
| Pooled Standard Deviation Estimates | | 17.5 | | 18.3 | | 1.1 | | 33.5 | |

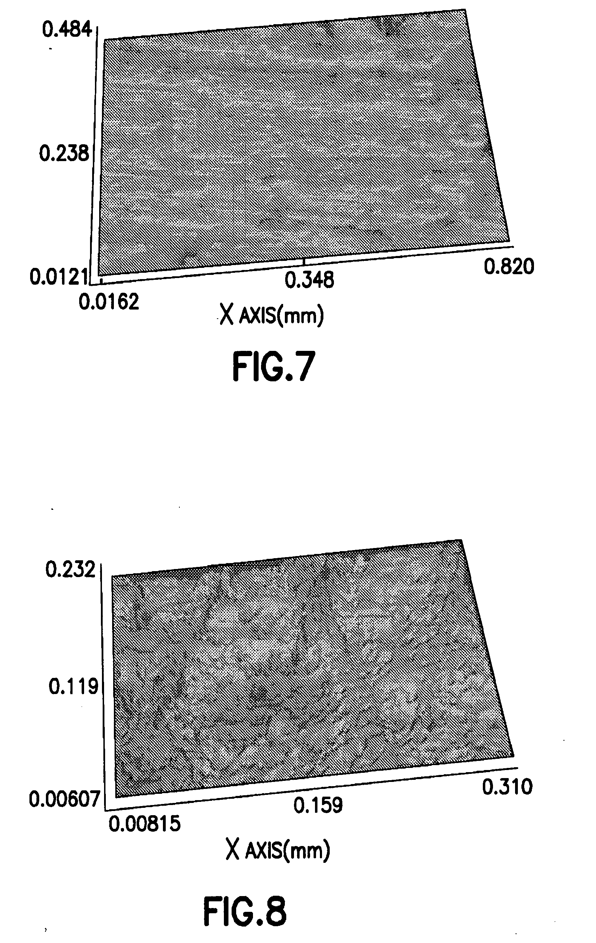

The swirling patterns as described may be the result of gas flow marks on the surface of the manufactured part. Surface defects, like swirling, represent one of the problems associated with the adoption of the process of using supercritical fluids to produce foamed parts. Such swirling is likely caused by bubbles at the surface being dragged against a mold wall. Referring to FIGS. 7 and 8, it can be seen that there are differences in surface profiles for conventional injection molded parts versus microcellular parts. Figure 7 shows a part molded by a conventional method, whereas FIG. 8 shows a part molded using a microcellular injection molding process.

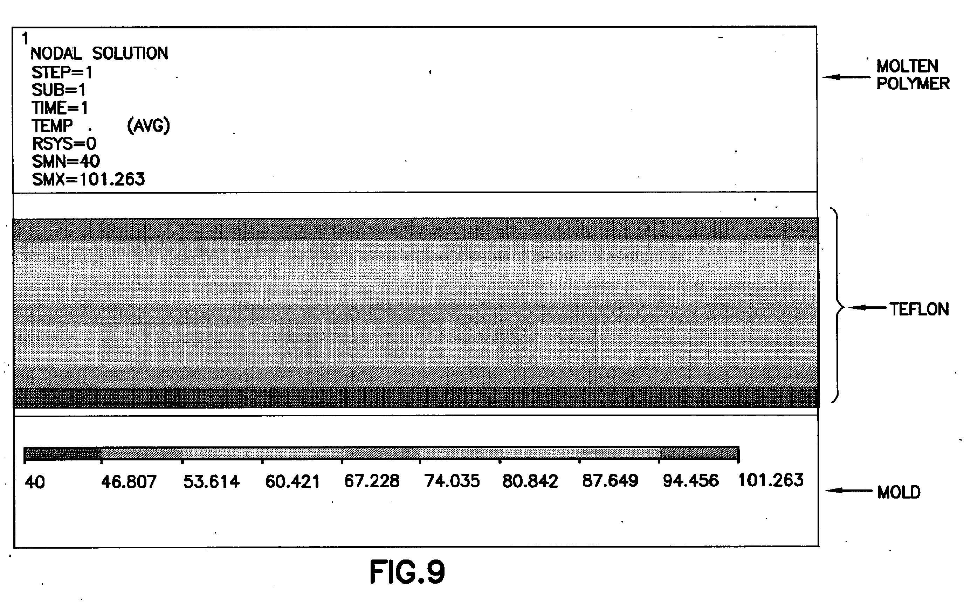

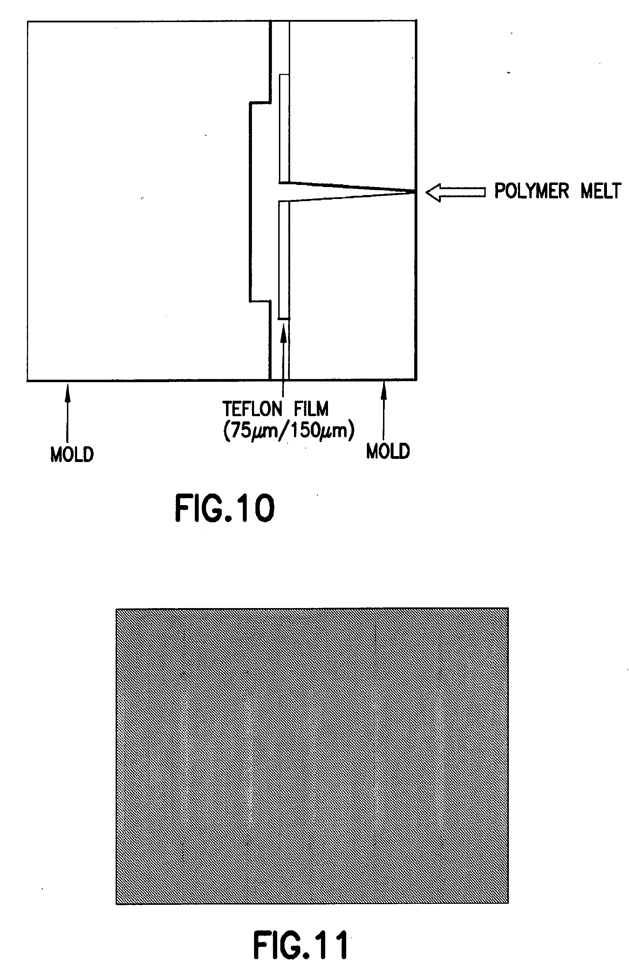

In one embodiment, which is not part of the present invention, by keeping the mold temperature high during injection by inserting an insulator (e.g., Teflon polytetrafluoroethylene (PTFE) or Teflon perfluoroalkoxy copolymer (PFA)) between the mold wall and the polymer, the surface quality of a part can be improved. To provide for the effective improvement in surface quality, simulations were done using a computer simulation program ANSYS 11.0 (available from Ansys, Lebanon, New Hampshire). Utilizing the thermal properties of polyethylene, stainless steel, PFA, and PTFE, temperatures were computed for the mold at various thicknesses of PFA and PTFE. Figure 9 provides the results of one such simulation in which the temperature at the wall is about 101 degrees C. These simulation results suggested experiments that could be done to improve the surface characteristics of tensile bars made using the supercritical fluid process. Figure 10 provides a schematic of the experiments that were performed.

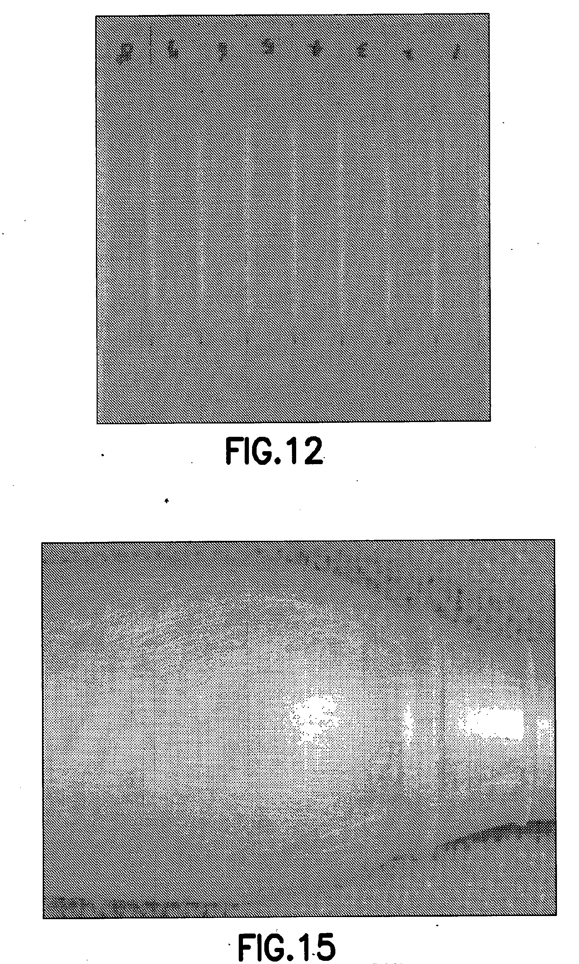

As a result of the computer simulation experiments, the surface was determined to be significantly improved with the use of 150 micron PTFE on the part thickness of 3.2mm. In particular, it was noted that swirl marks were eliminated or at least significantly reduced. However, it was also noted that with 75 micron PTFE, some samples continued to exhibit gas flow marks on the surface, while flow marks were eliminated for some samples, and in spite of a generally improved surface, some defects were still noted. Figures 11 and 12 illustrate some results of the parts made using PTFE coated molds. In particular, FIG. 11 shows a tensile and flexural test bar produced by the supercritical fluid process and using a 150 micron PTFE coating a on a mold, and FIG. 12 shows a tensile bar produce by the supercritical fluid process and using a 75 micron PTFE coating on a mold.

### Example 2 - Tampon applicator barrel experiment

A four-cavity hot-runner mold exhibiting significant part complexity was mechanically and electrically linked both to the Arburg 320S injection molding system, including the feed and conveying systems, as shown in FIG. 1. A rounded, radiused nozzle was used to inject LDPE plastic. Electrical zone heating for the hot runner manifold was controlled by means of a temperature controller (available from Gammaflux Inc., Sterling, Virginia). The mold was cooled using a chilled water system using inlet temperatures of either 10 or 22 degrees C. Other parameters were similar to those already described above for the tensile bar molding.

Some other molding parameters used in the tampon applicator molding include:
Core pull option set to activate prior to mold open and retracted prior to mold close
Air-actuated part ejection
Flow rates of 20-40 cubic centimeters per second
Barrel and molding temperatures of about 210 or 216 degrees C (410-420 degrees F)
Circumference speed of 13.0 meters per minute
Back pressure of 50-100 bar
Nitrogen dosage volume of 10.3 cubic centimeters
Supercritical fluid molding process: supercritical fluid delivery pressure maintained at about 1.4 MPA higher than melt pressure in the barrel to ensure nitrogen incorporation into the molded part
Injection fill time of 0.3-0.7 seconds
Pack time of 0 seconds
Cooling cycle time of 6-15 seconds
Overall cycle time of about 8-10 seconds longer than cooling time

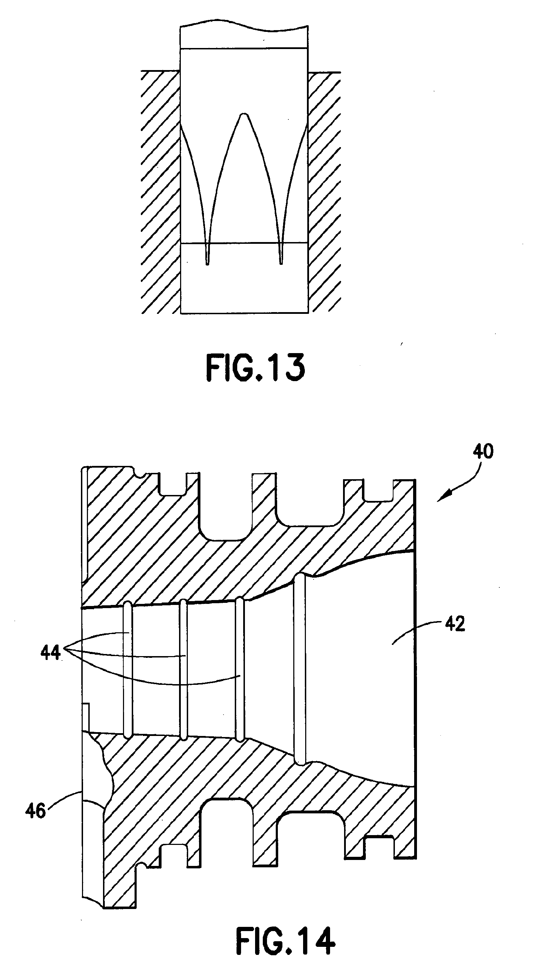

The parts produced from the process disclosed herein are shown in FIGS. 13 and 14 to illustrate the complexity with which parts can be produced using this process. In FIG. 13, a tampon applicator barrel petal end is shown. In FIG. 14, a mold for grip portion (a fingergrip) of a tampon applicator barrel is shown generally at 40. The tampon applicator barrel is shown at 42. Once molded, the tampon applicator barrel 42 includes raised ridges 44 that facilitate gripping by a user.

The grip portion 40 and the tampon applicator barrel 42 were molded over the course of a two-day period. The ratio of base resin to colorant used was 19 to 1 (as in the tensile bar molding experiment of Example 1).

In addition to the use of 100% LDPE, similar parts were manufactured using a composition of 95% LDPE with the balance being PTFE (namely, MP-1600 grade Teflon®). This composition was provided by E.I. DuPont Company of Wilmington, Delaware. One part in particular was manufactured for a feminine hygiene device using a composition of 95.5% LDPE, 3% green colorant mix, and 1.5% oleamide. This part exhibited a 6.4% weight reduction over other parts manufactured by the inventive microcellular injection molding process and demonstrated improved surface quality.

At least some of the parts were manufactured without colorant.

Samples were collected and tested in order to provide exemplification of the supercritical fluid process of the present invention for molding. The supercritical fluid process samples were denoted as E1, E2, E3, and E4. Comparative samples produced by conventional injection molding processes are denoted as C4 and C5. The samples are described in Table 4 below.

**Table 4 - Description of samples for molded tampon applicator barrel**

| Sample | Composition | Sample size (number of barrels) |
|---|---|---|
| E1 | 100% LDPE* | 13 |
| E2 | LDPE, no colorant | 6 |
| E3 | 95% LDPE*, 5% PTFE, 6 second cooling time | 23 |
| E4 | 95% LDPE*, 5% PTFE, 15 second cooling time | 23 |
| C4 | UW LDPE control, green colorant | 12 |
| C5 | 95% LDPE*, 5% PTFE | 9 |

| | | |
|---|---|---|
| * 95% LDPE + (5%)(0.73 LDPE) = 98.65% LDPE (actual amount), with the remaining 1.35% being colorant and other additives | | |

Results obtained in these trials were also compared with comparable tampon applicator barrel samples obtained from a large-scale conventional, commercial production-scale injection molding machine (hereinafter denoted as C6).

Some molding trials were also performed using a small percentage of nano-clay blends.

The following results were noted:

Due to slight processing differences, the C4 control sample parts were about 5% higher in weight as compared to the C6 parts.

The E1 parts were about 5% lower in part weight than the C4 parts.

The petals formed by the supercritical fluid process produced no flashing, and no other problems were noted (e.g., no problems were noted with closing of the petals in a tampon applicator).

Slight differences in surface quality were noted. In particular, barrel surfaces for the supercritical fluid process parts (E1-E4) were rougher than those for the comparison parts (C4-C6). Differences were less for those parts made using 5% PTFE, especially for the samples run at shorter cooling times.

Dimensional stability over time was determined to be suitable.

Table 5 below provides a summary of the measured weights and dimensions for samples C4 versus E1. Some differences, for example, the differences in fingergrip heights, may be considered to be not statistically significant.

**Table 5 - Summary of measured weights and dimensions**

| Attribute | Average of UW Control, C4 | Std Dev of UW Control, C4 | Average of LDPE, E1 | Std. Dev. of LDPE, E1 | % Decrease (Control, C4 to E1) |
|---|---|---|---|---|---|
| Fingergrip Inside Diameter | 0.255 | 0.0007 | 0.243 | 0.0071 | 4.52% |
| Fingergrip Length | 0.825 | 0.0015 | 0.825 | 0.0021 | 0.05% |
| Fingergrip Outside Diameter | 0.340 | 0.0016 | 0.322 | 0.0218 | 5.30% |
| Length from bottom to center of first finger grip ring | 0.225 | 0.0019 | 0.225 | 0.0019 | -0.03% |
| Length from bottom to center of second fingergrip ring | 0.426 | 0.0020 | 0.426 | 0.0028 | 0.02% |
| Length from bottom to center of third fingergrip ring | 0.628 | 0.0013 | 0.626 | 0.0044 | 0.25% |
| Length from parting line to highest petal tip | 1.848 | 0.0014 | 1.834 | 0.0038 | 0.77% |
| Middle ring raised height | 0.015 | 0.0008 | 0.017 | 0.0033 | -14.04% |
| Overall Barrel Length | 3.106 | 0.0036 | 3.092 | 0.0038 | 0.45% |
| Weights, grams | 3.419 | 0.0127 | 3.258 | 0.0371 | 4.71% |

The supercritical fluid process of the present invention forms micro-bubbles at the surface, such bubbles being dragged against the mold wall. The texture of these parts is somewhat "rougher," due at least in part to one or more of surface finish, cooling, and flow geometry. On the other hand, the inside of the barrel and fingergrip areas are more polished, thus these surface portions of the barrel feel less rough. The grit size is comparable to and may correspond to the presence of the micro-bubbles. This slightly "rougher" texture may be a positive consumer-relevant product feature. Without wishing to be bound by any particular theory, it is surmised that the presence of PTFE improves the surface "feel" of the part produced.

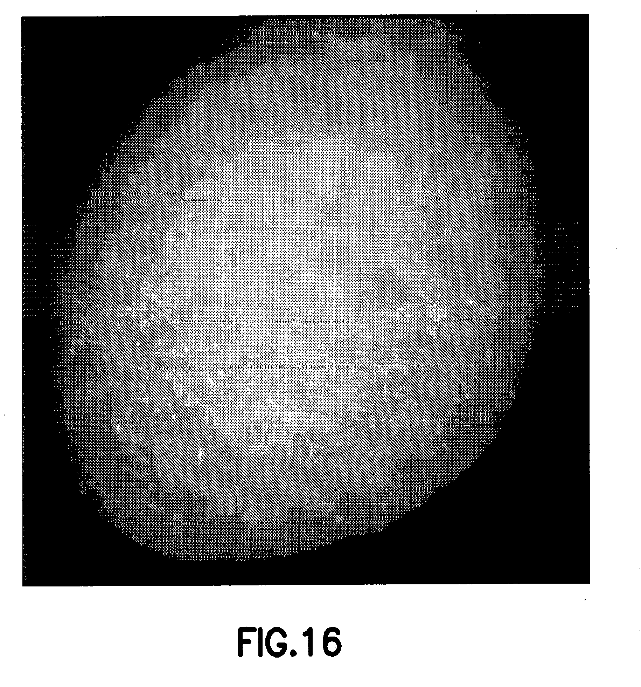

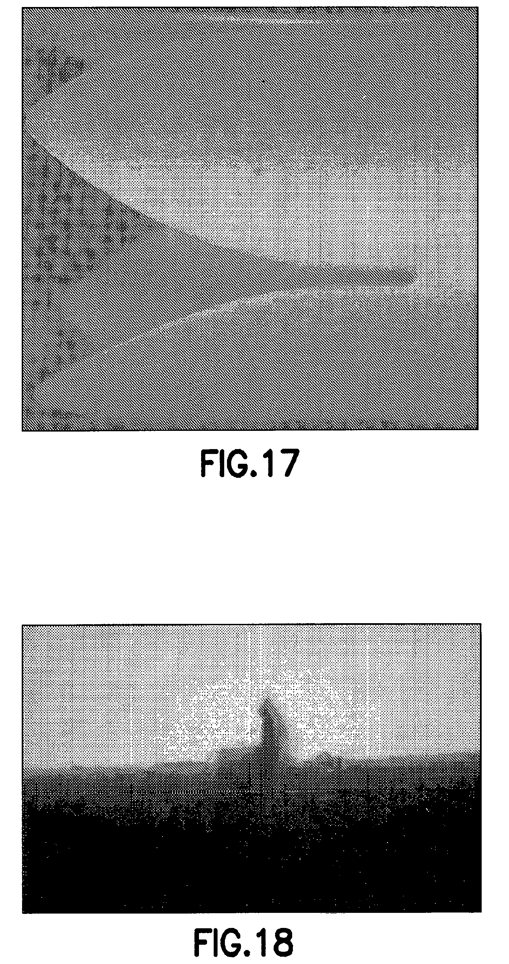

Referring now to FIGS. 15-18, various portions of the parts produced are shown. In particular, FIG. 15 shows a close-up of a surface of an applicator barrel formed by the process of the present invention; FIG. 16 shows the surface of a portion of a barrel formed by the process of the present invention at high magnification and through a rounded lens to show the bubble sizes and bubble distribution; FIG. 17 shows a close up view of the petals for parts made according to the present invention; and FIG. 18 shows a "shark-fin" geometry of a fingergrip at high magnification.

### Example 3: Additional Tampon Applicator Barrel Experiments

The same four-cavity hot-runner mold described in Example 2 above was used to make some additional tampon applicator barrels. In this particular four-cavity mold, two of the cavities were the same ones used for Example 2. The tampon applicator barrels made with these two cavities resulted in barrels which correspond to a tampon having more absorbency than a regular, average, or more widely used tampon (a "super absorbent" tampon). The other two barrels were made with barrels that were more slender; that is, smaller diameter barrels that are typically used for the smaller, regular, or lower absorbency tampons. The mold is configured to allow either sets of these two cavities to be used, but not all four at once. Moreover, the two cavities used for the regular or lower absorbency tampon barrels were diamond polished to provide a very smooth, polished finish. The cavities for the barrels corresponding to the more absorbent tampons were diamond polished. Several hundred tampon barrels were made for several sets of conditions; conditions which were roughly similar to those described in Example 2, except that shot sizes and temperature profiles were adjusted slightly in order to get lower part weights for the micro-cell-formed barrels. Conditions are provided in Table 6 below.

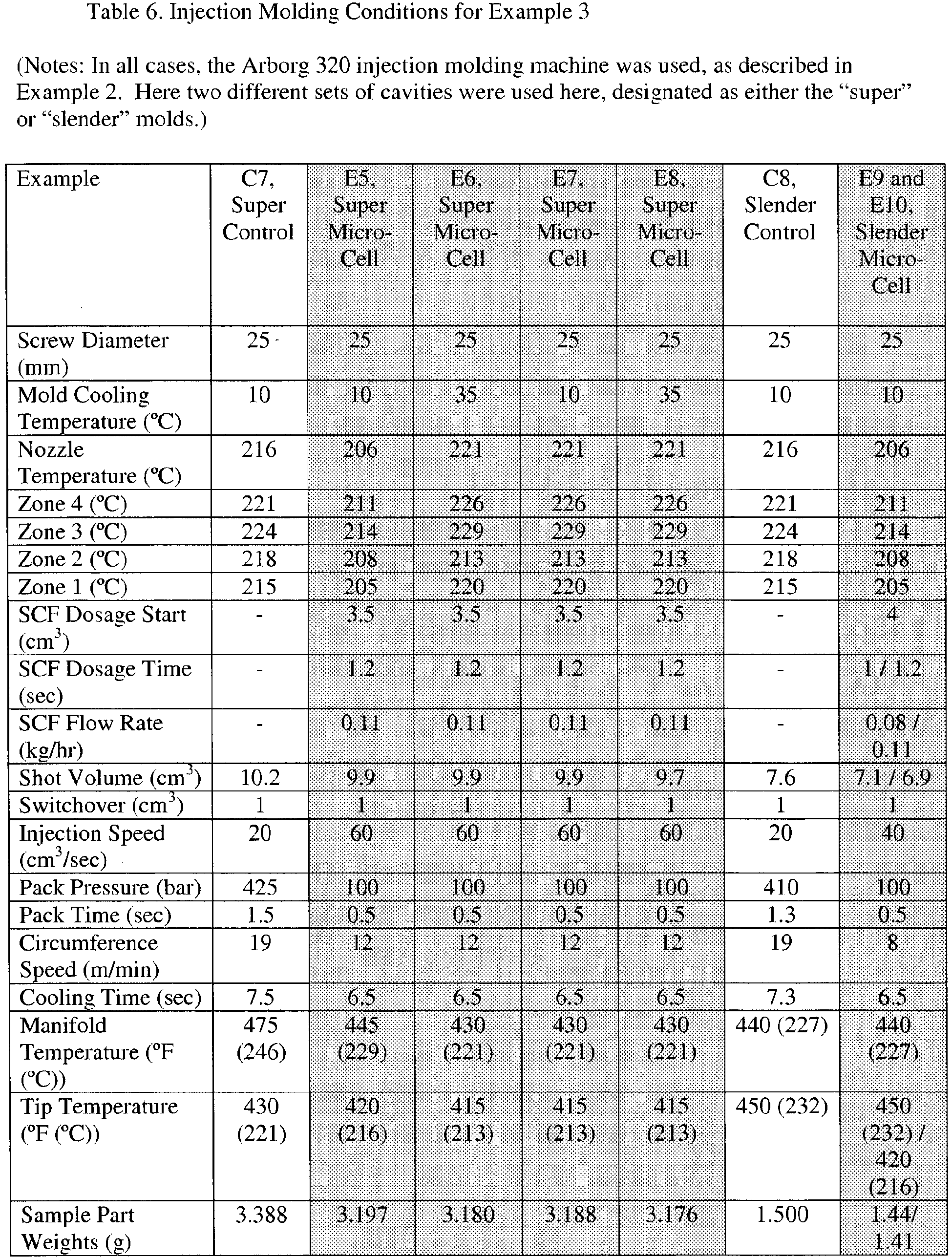

LDPE (Marlex KN226, Chevron-Phillips) was blended in a 19 to 1 ratio with the same green colorant formulation.)

Table 7 provides a summary of data collected for the barrels made in this Example. As you can see, the barrels formed by the micro-cell process, whether super (E5-E8) or slender (E9-E10), are lighter in weight than the corresponding control (C7 or C8) comparative barrels made by conventional injection molding using the same injection molding machine and the same molds. The attributes recorded here are averages taken from at least five different measurements. Pooled standard errors of estimate were also recorded here.

While the weights are clearly different for the micro-cell-produced barrels, the barrel lengths, petal thicknesses, petal gaps, fingergrip inside diameters, and fingergrip heights are roughly comparable to those made by conventional molding. This data suggests that even very thin parts - such as the parts at the tampon applicator petals, which are only 0.012 to 0.014 inches thick - can be made dimensionally stable using micro-cell technology. This is something of a surprise, given what has been taught to date in the prior art pertaining to this technology. More particularly, when the tampon applicator barrel petals for the more absorbent tampons were formed in a machine to soften, shape and close the petals in order to encapsulate the fibrous tampon pledget, the "petal gap" or average distance between the closed petals remains small, comparable to those measured for C7, which was molded by conventional injection molding.

For this Example, surface roughness was also measured. A simple, hand-held Federal Mahr (NJ) Pocket Surf II profileometer was used to make these measurements. These measurements were repeated multiple times (about 20 times for each sample listed here). The surface roughness for the slender barrels are less than those for the molds for the more absorbent tampons, likely because of the diamond polishing. But the average micro-cell slender roughness values (samples E9 and E10) were actually either comparable to or slightly less (i.e. more smooth) than those for the conventionally molded samples (C8). Roughness values for the more absorbent applicator barrels made by conventional molding (C7) were slightly lower than those for the comparably made micro-cell barrels (E5-E8).

**Table 7. Weights, Petal Thicknesses, and Other Key Dimensions for Tampon Applicator Barrels of Example 3**

| ***Example ID*** | ***Barrel Size*** | ***Type*** | ***Average. Weights, grams*** | ***Overall Barrel Length, avg., in.*** | ***Petal thickness, avg., in.*** | ***Surface roughness, avg., microns*** | ***Petal gap, avg., in.*** | ***Fingergrip Inside Diameter, avg., in.*** | ***Height of Middle Ring of Finger Grip, avg., in*** |
|---|---|---|---|---|---|---|---|---|---|
| C7 | Super | Control | 3.3879 | 3.0804 | 0.0123 | 0.7240 | 0.0460 | 0.2521 | 0.0175 |
| | | | | | | | | | |
| E5 | Super | Micro-Cell | 3.1973 | 3.0835 | 0.0126 | 1.1110 | 0.2067 | 0.2503 | 0.0143 |
| E6 | Super | Micro-Cell | 3.1621 | 3.0794 | 0.0129 | 0.9380 | 0.1256 | 0.2451 | 0.0146 |
| E7 | Super | Micro-Cell | 3.1729 | 3.0885 | 0.0127 | 0.9687 | 0.0262 | 0.2460 | 0.0167 |
| E8 | Super | Micro-Cell | 3.1674 | 3,0817 | 0.0126 | 0.8385 | 0.0438 | 0.2354 | 0.0139 |
| | | | | | | | | | |
| C8 | Slender | Control | 1.5014 | 2.9901 | 0.0132 | 0.0650 | | 0.2724 | 0.0127 |
| | | | | | | | | | |
| E9 | Slender | Micro-Cell | 1.4400 | 2.9837 | 0.0136 | 0.6095 | | 0.2810 | 0.0141 |
| E10 | Slender | Micro-Cell | 1.4186 | 2.9816 | 0.0139 | 0.5040 | | 0.2640 | 0.0146 |
| | | | | | | | | | |
| Pooled Standard Error Estimates | | | 0.0046 | 0.0030 | 0.0002 | 0.0304 | 0.0542 | 0.0020 | 0.0007 |

From the foregoing, it can be concluded that it is possible to make foam parts using the LDPE resin by a Microcellular injection molding process. Also, it can be concluded that a low melt temperature hinders cell nucleation, and that this hindrance is likely the result of an increase in the activation energy barrier. Higher melt temperatures increase the cell population density and decrease the cell size in the microcellular injection molded parts, possibly by lowering the activation energy for nucleation. However, excessively high melt temperatures and high weight reductions of the parts can result in low melt strengths, thereby causing cell coalescence and sometimes larger cells. Furthermore, microcellular injection molded parts can be produced with a weight reduction of up to 20% and exhibiting small bubbles ranging from 5 micrometers to 200 micrometers in size. Finally, using this microcellular process, it is possible to make dimensionally stable parts whose thickness can be as low as ten thousandths of an inch.

## Claims

1. A method of injection molding to produce a microcellular material, the method comprising the steps of:
melting a polymer, where the polymer is low-density polyethylene at a concentration of at least 70 wt.%;
blending said melted polymer with a supercritical fluid to produce a single-phase polymer-gas solution, wherein said supercritical fluid is nitrogen loaded at 0.04 wt.% to 1 wt.%;
maintaining said single-phase polymer-gas solution at a temperature greater than 10 centigrade degrees above the melting point of the polymer after the addition of said supercritical fluid and before the injection of said single-phase polymer-gas solution through said nozzle;
injecting said single-phase polymer-gas solution into a mold; and
causing said polymer to solidify thereby forming an article in said mold;
wherein the total cycle time of forming said material is 3 seconds to 100 seconds; and
wherein a pressure drop across a nozzle causes said supercritical fluid to nucleate gas bubbles in said polymer, thereby causing nucleation of a microcellular structure in said polymer, **Characterized in that** said polymer further comprises 0.1 wt.% to 20 wt.% of at least one of polytetrafluoroethylene and perfluoroalkoxy.

2. The method of claim 1, comprising a blend of at least 80 wt.% low density polyethylene, from 0.1-10 wt.% of at least one of polytetrafluoroethylene and perfluoroalkoxy.

3. The method of claim 1, wherein the polymer is low-density polyethylene at a concentration of 95 wt.% with the balance being polytetrafluoroethylene.

4. The method of claim 1, where the temperature is between 190 deg C and 230 deg C.

5. The method of claim 1, wherein said supercritical fluid is loaded at 0.15 wt.% to 0.45 wt.%.

6. The method of claim 1, wherein the total cycle time of forming said material is 3 seconds to 20 seconds.

7. The method of claim 1, further comprising incorporating a colorant into said polymer.

8. The method of claim 1, further comprising incorporating nanometer-sized clay particles into said polymer.

9. The method of claim 8, wherein the amount of nanometer-sized clay particles is up to 10 wt.% of the total weight of resin.

10. The method of claim 1, further comprising incorporating one or more of an additive such as an antioxidant, a lubricant, a slip agent, an aesthetic quality-enhancing agent, a pigment, an opacifier, an impact modifier, a filler, a clay, a flame retardant, an insulating material, and/or a processing aid into said polymer.

11. The method of any one of claims 1 to 10, wherein said material formed is a tampon applicator barrel (42).

## Patentansprüche

1. Ein Spritzgießverfahren um ein mikrozellulares Material herzustellen, das Verfahren aufweisend die Schritte von:
Schmelzen eines Polymers, wobei das Polymer Polyethylen von geringer Dichte bei einer Konzentration von zumindest 70 Gew. % ist;
Mischen des geschmolzenen Polymers mit einem überkritischen Fluid, um eine Einphasen-Polymer-Gas-Lösung herzustellen, wobei das überkritische Fluid zu 0,04 Gew.% bis 1 Gew. % mit Stickstoff angereichert ist;
Aufrechterhalten der Einphasen-Polymer-Gas-Lösung bei einer Temperatur größer als 10 Grad Celsius über dem Schmelzpunkt des Polymers nach der Hinzugabe von dem überkritischen Fluid und vor der Einspritzung von der Einphasen-Polymer-Gas-Lösung durch die Düse;
Einspritzen der Einphasen-Polymer-Gas-Lösung in eine Gießform; und
Bewirken, dass das Polymer sich festigt und dabei einen Gegenstand in der Gießform formt;
wobei die gesamte Zykluszeit der Materialformung 3 Sekunden bis 100 Sekunden ist; und
wobei ein Druckabfall über einer Düse bewirkt, dass das überkritische Fluid Gasbläschen in dem Polymer keimbildet,
dabei Bewirken einer Keimbildung einer mikrozellularen Struktur in dem Polymer, **dadurch gekennzeichnet, dass** das Polymer weiter aufweist 0,1 Gew.% bis 20 Gew.% von zumindest einem von Polytetrafluorethylen und Perfluoralkoxy.

2. Das Verfahren gemäß Anspruch 1, aufweisend eine Mischung von zumindest 80 Gew.% Polyethylen von geringer Dichte, von 0,1 - 10 Gew.% von zumindest einem von Polytetrafluorethylen und Perfluoralkoxy.

3. Das Verfahren gemäß Anspruch 1, wobei das Polymer Polyethylen von geringer Dichte bei einer Konzentration von 95 Gew.% ist und der Rest Polytetrafluorethylen ist.

4. Das Verfahren gemäß Anspruch 1, wobei die Temperatur zwischen 190 Grad C und 230 Grad C ist.

5. Das Verfahren gemäß Anspruch 1, wobei das überkritische Fluid zu 0,15 Gew.% bis 0,45 Gew.% angereichert ist.

6. Das Verfahren gemäß Anspruch 1, wobei die gesamte Zykluszeit der Materialformung 3 Sekunden bis 20 Sekunden ist.

7. Das Verfahren gemäß Anspruch 1, weiter aufweisend Einbinden eines Farbstoffs in das Polymer.

8. Das Verfahren gemäß Anspruch 1, weiter aufweisend Einbinden nanometergroßer Tonpartikel in das Polymer.

9. Das Verfahren gemäß Anspruch 8, wobei die Menge an nanometergroßen Tonpartikeln bis zu 10 Gew.% des Gesamtgewichts des Granulats ausmacht.

10. Das Verfahren gemäß Anspruch 1, weiter aufweisend Einbinden eines oder mehrerer von einem Additiv wie etwa einem Antioxidationsmittel, einem Schmiermittel, einem Gleitmittel, einem die ästhetische Qualität steigernden Mittel, einem Pigment, einem Trübungsmittel, einem Schlagzähmodifizierer, einem Füllstoff, Ton, einem Flammschutzmittel, einem Dämmstoff und/oder einem Verarbeitungshilfsstoff in das Polymer.

11. Das Verfahren gemäß einem der Ansprüche 1 bis 10, wobei das Material, welches geformt wird, ein Tampon-Applikator-Gehäuse (42) ist.

## Revendications

1. Procédé de moulage par injection pour produire un matériau microcellulaire, le procédé comprenant les étapes consistant à :
faire fondre un polymère, le polymère étant du polyéthylène à faible densité à une concentration d'au moins 70 % en poids ;
mélanger ledit polymère fondu avec un fluide supercritique pour produire une solution polymère-gaz à phase unique, ledit fluide supercritique étant de l'azote chargé de 0,04 % en poids à 1 % en poids ;
maintenir ladite solution polymère-gaz à phase unique à une température supérieure à 10 degrés centigrades au-dessus du point de fusion du polymère après l'ajout dudit fluide supercritique et avant l'injection de ladite solution polymère-gaz à phase unique à travers ladite buse ;
injecter ladite solution polymère-gaz à phase unique dans un moule ; et
faire solidifier ledit polymère pour ainsi former un article dans ledit moule ;
le temps de cycle total de la formation dudit matériau étant de 3 secondes à 100 secondes ; et une chute de pression à travers une buse entraînant une nucléation des bulles de gaz dans ledit polymère par ledit fluide supercritique, entraînant ainsi la nucléation d'une structure microcellulaire dans ledit polymère, **caractérisé en ce que** ledit polymère comprend en outre 0,1 % en poids à 20 % en poids de polytétrafluoroéthylène et/ou perfluoroalkoxy.

2. Procédé selon la revendication 1, comprenant un mélange d'au moins 80 % en poids de polyéthylène à faible densité, de 0,1 à 10 % en poids de polytétrafluoroéthylène et perfluoroalkoxy.

3. Procédé selon la revendication 1, dans lequel le polymère est du polyéthylène à faible densité à une concentration de 95 % en poids, le reste étant du polytétrafluoroéthylène.

4. Procédé selon la revendication 1, dans lequel la température se situe entre 190 °C et 230 °C.

5. Procédé selon la revendication 1, dans lequel ledit fluide supercritique est chargé de 0,15 % en poids à 0,45 % en poids.

6. Procédé selon la revendication 1, dans lequel le temps de cycle total de la formation dudit matériau est de 3 secondes à 20 secondes.

7. Procédé selon la revendication 1, consistant en outre à incorporer un colorant dans ledit polymère.

8. Procédé selon la revendication 1, consistant en outre à incorporer des particules d'argile de taille nanométrique dans ledit polymère.

9. Procédé selon la revendication 8, dans lequel la quantité de particules d'argile de taille nanométrique est au maximum de 10 % en poids du poids total de la résine.

10. Procédé selon la revendication 1, consistant en outre à incorporer un ou plusieurs additifs tels qu'un antioxydant, un lubrifiant, un agent anti-adhérent, un agent améliorant les qualités esthétiques, un pigment, un opacifiant, un agent modifiant la résistance aux chocs, un agent de remplissage, de l'argile, un retardateur de flamme, un matériau isolant et/ou un adjuvant de fabrication dans ledit polymère.

11. Procédé selon l'une quelconque des revendications 1 à 10, dans lequel ledit matériau formé est un cylindre d'applicateur de tampon (42).
